# EUROPEAN PATENT APPLICATION

(11) **EP 4 501 971 A1**
(43) Date of publication of application: **05.02.2025**
(21) Application number: 23215324.7
(22) Date of filing: 08.12.2023
(51) Int. Cl.: C08F 2/44, C08F 212/08, C08F 220/14, C08F 212/36, C08F 222/10, C08F 2/24, C08J 3/20, C08K 5/01, C08K 5/54, C09K 11/06, G01N 33/53, B01J 3/00

(54) **RESIN PARTICLE COMPOSITION, TEST KIT, AND MANUFACTURING METHOD OF RESIN PARTICLE COMPOSITION**

(30) Priority: 04.08.2023 JP 2023127972
(71) Applicant: Saiden Chemical Industry Co., Ltd., Tokyo 103-0023 (JP)
(72) Inventor: KANEDAI, Shuichi, Saitama (JP); KIJIMA, Kenji, Saitama (JP)
(74) Representative: Drywood, Rosalind Aerona

(57) **Abstract**

There is provided with a resin particle composition containing resin particles incorporating a marker substance. The resin particle composition is manufactured by a method comprising generating a mixture by mixing the marker substance and a polymerizable monomer using subcritical water (100, 200), generating a monomer particle dispersion liquid by mixing and cooling the mixture and an emulsifier (300, 400), and generating the resin particles by causing a polymerization reaction between the monomer particle dispersion liquid and a crosslinkable monomer.

## Description

### BACKGROUND OF THE INVENTION

### Field of the Invention

The present invention relates to a resin particle composition, a test kit, and a manufacturing method of the resin particle composition.

### Description of the Related Art

It is recognized that a resin particle composition containing a marker substance is useful as a fluorescent marker in, for example, a clinical examination in the biology/medical diagnostic field or the like. Also, according to marker substance type, a marker substance is used in a diluted state or a high concentration state in which the substance is evenly dispersed in a polymeric host material or solvent in a molecular level. For example, there is disclosed a method of granulating polymer particles under the existence of a fluorescent dye and causing the polymer particles to incorporate the fluorescent dye in the polymer granulation process (Japanese Patent No. 6978081). There is also disclosed a method of swelling polymer particles in a liquid containing a fluorescent dye, thereby causing the polymer particles to incorporate the fluorescent dye (Japanese Patent No. 6769728).

However, there is a problem that when monomer oil droplets are being made small using a high-shear dispersion apparatus such as a high-pressure homogenizer, the dispersion liquid becomes hot, and the dissolved fluorescent dye precipitates, and therefore, the amount of fluorescent dye that the polymer particles can incorporate is limited (Japanese Patent No.6978081). There is also a problem that since the fluorescent dye incorporating particles produced using the polymer particles that are readily swollen in a liquid are dissolved by a solvent, the fluorescence intensity is low (Japanese Patent No. 6769728). Thus, a resin particle composition having an excellent solvent resistance and a high fluorescence intensity cannot be implemented.

### SUMMARY OF THE INVENTION

The present invention provides a resin particle composition having an excellent solvent resistance and a high fluorescence intensity.

The present invention in its one aspect provides a resin particle composition as specified in claims 1 to 6.

The present invention in its one aspect provides a test kit as specified in claim 7.

The present invention in its one aspect provides a manufacturing method of a resin particle composition as specified in claim 8.

Further features of the present invention will become apparent from the following description of exemplary embodiments (with reference to the attached drawings).

### BRIEF DESCRIPTION OF THE DRAWINGS

Figure is a view for explaining a manufacturing step of a monomer particle dispersion liquid using a supercritical water supply device.

### DESCRIPTION OF THE EMBODIMENTS

Hereinafter, embodiments will be described in detail with reference to the attached drawings. Note that the following embodiments are not intended to limit the scope of the claimed invention, and limitation is not made an invention that requires all combinations of features described in the embodiments. Two or more of the multiple features described in the embodiments may be combined as appropriate. Furthermore, the same reference numerals are given to the same or similar configurations, and redundant description thereof is omitted.

### (Resin Particle Composition)

A resin particle composition according to one embodiment contains resin particles incorporating a marker substance. The resin particle composition according to the embodiment is manufactured by a method comprising generating a mixture by mixing the marker substance and a polymerizable monomer using subcritical water, generating a monomer particle dispersion liquid by mixing and cooling the mixture and an emulsifier, and generating the resin particles by causing a polymerization reaction between the monomer particle dispersion liquid and a crosslinkable monomer.

### (Resin Particles)

The polymerizable monomer according to the embodiment contains at least one of an aromatic monomer, a (meth)acrylic monomer, and a monomer containing a functional group. The resin particles according to the embodiment contain 40 to 95 mass% of the aromatic monomer and/or the (meth)acrylic monomer, 0 to 10 mass% of the monomer containing the functional group, and 5 to 60 mass% of the crosslinkable monomer as constituent units with respect to 100 mass% of a total polymerizable monomer. Here, "100 mass% of a total polymerizable monomer" represents mass% of the total polymerizable monomer that forms the resin particles, and represents the total mass% of the polymerizable monomer and the crosslinkable monomer. Also, "40 to 95 mass% of the aromatic monomer and/or the (meth)acrylic monomer" indicates that three types of forms (40 to 95 mass% of the aromatic monomer, 40 to 95 mass% of the (meth)acrylic monomer, and 40 to 95 mass% of a mixture of the aromatic monomer and the (meth)acrylic monomer) are included.

The average particle size of the resin particles according to the embodiment is 50 to 500 nm. Here, the average particle size is measured by a dynamic light scattering method.

A polymerizable monomer (monomer), an emulsifier, and a polymerization initiator, which can be used in the manufacturing method of the resin particles according to the present invention, will be described. Note that a term "(meth)acryl" in this specification means both "acryl" and "methacryl", and a term "(meth)acrylate" means both "acrylate" and "methacrylate".

Polymerizable monomers usable for manufacturing of the resin particles will be described as an example. However, the monomers are not limited to these. A single one of the monomers below or a mixture of two or more of them can be used.

The aromatic monomer is not particularly limited if it dissolves the marker substance, and includes at least one substance selected from the group consisting of styrene, methylstyrene, chlorostyrene, methoxystyrene, α-methylstyrene, p-nitrostyrene, ethylvinylbenzene, vinylnaphthalene, benzyl acrylate, benzyl methacrylate, phenylethyl acrylate, phenylethyl methacrylate, phenylpropyl acrylate, phenylpropyl methacrylate, phenylnonyl acrylate, and phenylnonyl methacrylate.

The content of the aromatic monomer according to the embodiment is preferably 40 mass% or more, and more preferably 40 to 95 mass% with respect to 100 mass% of the total polymerizable monomer.

Examples of the (meth)acrylic monomer are alkyl (meth)acrylates whose number of carbon atoms is 1 to 18 such as methylethyl (meth)acrylate, ethyl (meth)acrylate, n-propyl (meth)acrylate, i-propyl (meth)acrylate, n-butyl (meth)acrylate, i-butyl (meth)acrylate, sec-butyl (meth)acrylate, t-butyl (meth)acrylate, n-amyl (meth)acrylate, i-amyl (meth)acrylate, n-hexyl (meth)acrylate, 2-ethylhexyl (meth)acrylate, n-octyl (meth)acrylate, nonyl (meth)acrylate, decyl (meth)acrylate, dodecyl (meth)acrylate, lauryl (meth)acrylate, and stearyl (meth)acrylate; and cycloalkyl (meth)acrylates such as cyclohexyl (meth)acrylate and p-t-butylcyclohexyl (meth)acrylate.

The content of the (meth)acrylic monomer according to the embodiment is preferably 40 mass% or more, and more preferably 40 to 95 mass% with respect to 100 mass% of the total polymerizable monomer.

As the crosslinkable monomer, at least one substance selected from the group consisting of allyl methacrylate, ethyleneglycol dimethacrylate, ethyleneglycol diacrylate, butanediol diacrylate, butanediol dimethacrylate, neopentyl glycol dimethacrylate, hexanediol dimethacrylate, triethyleneglycol dimethacrylate, tetraethyleneglycol dimethacrylate, trimethylolpropane trimethacrylate, pentaerythritol tetramethacrylate, and divinylbenzene can be used. As the crosslinkable monomer, divinylbenzene that is an aromatic material is particularly preferable.

The content of the crosslinkable monomer according to the embodiment is preferably 5 to 60 mass%, 5 to 50 mass%, or 5 to 40 mass%, and more preferably 5 to 30 mass% with respect to 100 mass% of the total polymerizable monomer. If the resin particles contain the crosslinkable monomer in the predetermined mass%, the solvent resistance of the resin particles can further be increased, and swelling of the resin particles in a solvent can be suppressed. The solvent resistance means the difficulty of the marker substance eluding to the outside of the resin particles.

Examples of the monomer containing the functional group are carboxyl group-containing polymerizable monomers such as monomers obtained by causing maleic anhydride, succinic anhydride, and phthalic anhydride to react with hydroxyalkyl (meth)acrylate such as (meth)acrylic acid, acrylic acid dimer, 2-hydroxyethyl (meth)acrylate, and 4-hydroxybutyl (meth)acrylate; hydroxyalkyl (meth)acrylates such as hydroxyethyl methacrylate, 2-hydroxyethyl (meth)acrylate, 2-(3-)hydroxypropyl (meth)acrylate, 4-hydroxybutyl (meth)acrylate, and glycerol mono(meth)acrylate; aminoalkyl (meth)acrylates such as 2-aminoethyl (meth)acrylate, 2-dimethylaminoethyl (meth)acrylate, 2-aminopropyl (meth)acrylate, and 2-butyl aminoethyl(meth)acrylate; and amide group-containing (meth)acrylates such as (meth)acrylamide, N-methylol acrylamide, and N-butoxymethyl (meth)acrylamide.

The content of the monomer containing the functional group according to the embodiment is preferably 0 to 10 mass% with respect to 100 mass% of the total polymerizable monomer. Here, in a case where the resin particles are used as antigen test light-emitting particles or antibody test light-emitting particles to be described later, the monomer containing the functional group can be used to bind the resin particles to a marker substance such as an antibody or antigen. Hence, if the resin particles according to the present invention are not used as the antigen test light-emitting particles or the antibody test light-emitting particles, the monomer containing the functional group may not be an essential component for manufacturing the resin particles.

The functional group according to the embodiment includes at least one of a hydroxy group, a carboxy group, an amino group, and an amide group.

### (Marker substance)

The content of the marker substance according to the embodiment is preferably 0.1 to 15 mass%, and more preferably 0.5 to 10 mass% with respect to 100 mass% of the total polymerizable monomer.

The marker substance according to the embodiment includes at least one of a fluorescent dye and an aggregation-induced emission dye (to be also referred to as an AIE dye hereinafter).

The fluorescent dye is a dye that emits fluorescent light. If the fluorescent dye absorbs light, electrons in the dye are excited to emit excess energy as an electromagnetic wave when returning to the ground state. The electromagnetic wave is fluorescent light, and the wavelength of the fluorescent light is always longer than the wavelength of the absorbed light (excited light). The difference between the wavelength of the excited light and the wavelength of the fluorescent light is called a stokes shift. If the stokes shift is large, the fluorescent light of the dye can readily be identified in distinction from the excited light and easily detected.

Examples of the fluorescent dye are compounds and derivatives having a fluorescent characteristic such as merocyanine, perylene, acridine, anthracene, luciferin, pyranine, stilbene, rhodamine, coumarin, 4-(dicyanomethylene)-2-methyl-6-(4-dimethyl aminostyryl)-4H-pyran (DCM), pyrromethene, fluorescein, and umbelliferone, and at least one substance selected from the group is usable.

The AIE dye is an aggregation-induced emission dye and is a fluorescent dye that does not emit light in a diluted solution state but emits strong light in a solid/aggregation state, and exhibits a behavior opposite to that of a general fluorescent dye. That is, the AIE dye is a dye capable of implementing a high concentration and high luminance, which are difficult in a normal fluorescent dye due to concentration quenching.

Examples of the AIE dye are silole ring-containing compounds and derivatives such as 1,1-dimethyl-2,3,4,5-tetraphenylsilole, 1,1,2,3,4,5-hexaphenylsilole, 1,1-dimethyl-2,5-dianisyl-3,4-diphenylsilole, and 1,1-diallyl-2,3,4,5-tetraphenylsilole, hydrocarbon aromatic-based compounds and derivatives such as tetraphenyl ethylene, heteroaromatic-based compounds and derivatives, and rhodamine-based compounds and derivatives, and at least one substance selected from the group is usable. In particular, a silole ring-containing compound is preferably used as the AIE dye from the viewpoint that it can easily be captured by the resin particles.

### (Polymerization Initiator)

The polymerization initiator is not particularly limited, and any one of conventionally known polymerization initiators can be used. For example, polymerization initiators to be described below, which are generally used for radical polymerization, can appropriately be used.

Examples of the polymerization initiator are persulfates such as potassium persulfate, sodium persulfate, and ammonium persulfate; oil-soluble azo compounds such as 2,2'-azobisisobutyronitrile, 2,2'-azobis(2-methyl butyronitrile), 2,2'-azobis(2,4-dimethyl valeronitrile), 2,2'-azobis(4-methoxy-2,4-dimethyl valeronitrile), and 2-phenyl azo-4-methoxy-2,4-dimethyl valeronitrile; water-soluble azo compounds such as 2,2'-azobis{2-methyl-N-[1,1-bis(hydroxymethyl)-2-hydroxyethyl]propionamide}, 2,2'-azobis{ 2-methyl-N-[2-(1-hydroxyethyl)]propionamide}, 2,2'-azobis{2-methyl-N-[2-(1-hydroxybutyl)]propionamide}, 2,2'-azobis[2-(5-methyl-2-imidazoline-2-yl)propane] and salts thereof, 2,2'-azobis[2-(2-imidazoline-2-yl)propane] and salts thereof, 2,2'-azobis[2-(3,4,5,6-tetrahydropyrimidine-2-yl)propane] and salts thereof, 2,2'-azobis{2-[1-(2-hydroxyethyl)-2-imidazoline-2-yl]propane} and salts thereof, 2,2'-azobis(2-methyl propionamidine) and salts thereof, 2,2'-azobis(2-methyl propyneamidine) and salts thereof, and 2,2'-azobis[N-(2-carboxyethyl)-2-methyl propionamidine] and salts thereof; and organic peroxides such as benzoyl peroxide, cumene hydroperoxide, t-butyl hydroperoxide, t-butyl peroxy-2-ethyl hexanoate, and t-butylperoxy isobutyrate.

### (Emulsifier)

The emulsifier according to the embodiment includes a nonionic emulsifier having a cloud point.

The cloud point of the nonionic emulsifier means a temperature at which, in a system formed by dispersing inorganic or organic particles in an aqueous medium, clouding occurs in the dispersion system in a heating process under the existence of the nonionic emulsifier. The cloud point can be measured under each reaction condition because it is influenced by the concentration of the nonionic emulsifier or an electrolyte. The cloud point of the nonionic emulsifier is, for example, 1°C to 99°C, preferably 20°C to 95°C, and more preferably 50°C to 90°C. Also, the hydrophilic-lipophilic balance (HLB) of the nonionic emulsifier can be selected in a wide range, and is, for example, 1 to 20, preferably 5 to 18, and more preferably about 8 to 16.

The nonionic emulsifier is not particularly limited if it satisfies the above-described conditions. Examples include polyoxyethylene alkyl ethers [polyoxyethylene C6-20 alkenyl ethers such as polyoxyethylene lauryl ether (available from Kao, EMULGEN 109P, cloud point 83°C, HLB 13.6), polyoxyethylene isodecyl ether (available from DKS, NOIGEN SD-80, cloud point 80°C, HLB 14.3), polyoxyalkylene branched decyl ether (available from DKS, NOIGEN XL-100, cloud point 79°C, HLB 14.7), polyoxyethylene tridecyl ether (available from DKS, NOIGEN TDS-80, cloud point 60°C, HLB 13.3), polyoxyalkylene tridecyl ether (available from DKS, NOIGEN TDX-50, cloud point 37°C, HLB 9.0), and polyoxyethylene oleyl ether (available from Kao, EMULGEN 409PV, cloud point 55°C, HLB 12.0)], [polyoxyethylene styrenated phenyl ether (available from DKS, NOIGEN EA-137, cloud point 65°C, HLB 13.0)], and [polyoxyethylene C6-20 alkylene alkenyl ether containing at least one ethylenically unsaturated group (ethylenically unsaturated compound) such as an allyl group (available from Kao, LATEMUL PD-420, cloud point 83°C, HLB 12.6), (available from Kao, LATEMUL PD-430, cloud point 95°C, HLB 14.4)]. These nonionic emulsifiers can be used solely or in a combination of two or more types.

### <Application Purposes>

A test kit according to the embodiment includes a resin particle composition that binds to a marker substance in a specimen. Note that since the resin particle composition according to the present invention described above can be used as the resin particle composition of the test kit, a detailed description of the resin particle composition will be omitted.

Application purposes of the resin particle composition according to the present invention are an organic EL light-emitting dye, a solar cell wavelength conversion dye, bioimaging, and the like. In particular, when the surface of the resin particle composition is modified with an antibody, it is useful as marker light-emitting particles by immunochromatography for an antigen test to be described later. More specifically, a resin particle composition with an antibody can suitably be used as antigen test light-emitting particles serving as a quick and easy test kit for COVID-19. Also, the resin particle composition with an antigen can suitably be used as antibody test light-emitting particles. Presently, gold colloid-based particles are used as the antigen test light-emitting particles. However, since the luminous sensitivity is low, the detection sensitivity is very low at an initial stage of infection where the number of viruses is small. That is, a patient suspected to have virus infection takes the PCR test in a hospital. Also, recently, the cycle of virus mutation is very short, and explosive spread of virus infection increases burden on medical institutions and patients. Based on such a backdrop, market needs for high-sensitivity antigen test kits that are easily available in pharmacies and drug stores are increasing, and the present invention can provide a resin particle composition having fluorescence sensitivity satisfying such needs.

### (Antigen Test)

An antigen test is a method of permeating a channel formed by a fiber such as cellulose with a liquid mixture of a specimen and a detection reagent and observing the appearance of a color line indicating that a marker substance (an antigen or an antibody) in the specimen and the detection reagent have reacted, thereby detecting the presence/absence of the marker substance, and an antigen test kit is widely used to easily and quickly detect an antigen. The antigen test is a test for detecting an antigen that is produced when a cell of a body is infected with a virus, and a pharynx wiping liquid is used as a specimen. When the pharynx wiping liquid is set in the test kit, a result is shown in about 15 min.

The antigen test is very easy and requires low cost as compared to the PCR test. However, the target to be detected cannot be amplified, unlike the PCR test. For this reason, if the test is performed at an early onset stage when the virus amount is small, correct detection may be impossible. Presently, a test result in the second to ninth days after symptoms appear is said to be correct, and it is considered that if it is tested negative during this period, COVID-19 infection can be denied. Although it is clear that the antigen test is useful, the antigen test of COVID-19 is presently considered as a screening test because of the problem of sensitivity. The screening test is executed for the purpose of specifying a person who may be infected, and an infection measure can be taken by this.

### (Immunochromatography)

In immunochromatography, (1) a quick test is possible because the time required until determination is short, (2) measurement can be done only by dropping a specimen, and the operation is easy, and (3) no special detection device is required, and determination is easy. Using these features, the immunochromatography is used not only for COVID-19 tests, influenza tests, pregnancy tests, and medical systems but also for food tests and environmental tests, and is widely used as a new Point Of Care Testing (POCT) method. Thus, the immunochromatography enables emergency tests in a hospital and tests during a surgery, and also tests at home outside a hospital, and the needs of this test method are particularly very large to novel viruses such as COVID-19.

The immunochromatography will be described in more detail. When a specimen containing an antigen is dropped to the specimen drop portion of a reagent device, the specimen permeates a conjugate pad and flows from the pad to a membrane filter by a capillary phenomenon while dissolving a marker antibody. The conjugate pad is made of, for example, glass fiber, polyester, or rayon and holds the marker antibody in a dry state. The antigen in the specimen binds to the marker antibody, thereby forming an immune complex. The immune complex of "marker antibody - antigen", which moves in the membrane filter by the capillary phenomenon, further binds to a capture antibody linearly immobilized on the membrane to form a complex of "marker antibody - antigen - capture antibody", and trapped to be blocked on the membrane. By this trap, light-emitting fine particles derived from the marker antibody are concentrated on the capture antibody line, and the color can be determined visually.

### <Manufacturing Method of Resin Particle Composition>

The manufacturing method of the resin particle composition according to the embodiment includes (i) a step of generating a mixture by mixing a marker substance and a polymerizable monomer using subcritical water, (ii) a step of generating a monomer particle dispersion liquid by mixing and cooling the mixture and an emulsifier, and (iii) a step of generating resin particles by causing a polymerization reaction between the monomer particle dispersion liquid and a crosslinkable monomer.

The monomer particle dispersion liquid will also be referred to as "oil droplets" or "microparticles" hereinafter. Here, in general, to disperse, as microparticles, the oil droplets (monomer particle dispersion liquid) obtained by dissolving the marker substance, micronization techniques using a high-pressure homogenizer or an ultrasonic homogenizer are known. However, these existing micronization techniques require a physical large force (for example, a shearing force or a destructive force by cavitation) at the time of stirring dispersion. It has been confirmed that at the time of stirring dispersion, precipitation of the marker substance to the outside of the oil droplets increases. The present inventor found that a subcritical water emulsification technology is used for the purpose of suppressing precipitation of the marker substance to the outside of the oil droplets. The outline of an existing micronization technique will be described below.

### (Existing Micronization Technique)

(1) Rotary stirring emulsification: there exist a propeller mixer, a homomixer, and a disper that are emulsification apparatuses using a general-purpose mechanical stirring technique combined with a stirring jig (a stirring blade, a turbine, or the like). These include a rotary stirring structure and is used for general emulsion manufacturing. With a batch method, the oil droplet particles in a micron size can be prepared by a stirring time.
(2) Ultrasonic homogenizer emulsification: emulsification is promoted using cavitation generated by an ultrasonic wave. With a batch method, the oil droplet particles in a submicron size can be prepared by a processing time.
(3) High-pressure homogenizer emulsification: a liquid is passed to a thin channel using a high-pressure pump, or a shearing force is given to liquids by making the liquids counter-collide against each other. This method generates a high emulsifying force and is therefore suitable for micronization of droplets. Oil droplet particles in a submicron size can be prepared by repeating the pass count by a continuous flow method. However, the repetitive operation is cumbersome.

A manufacturing method of a resin particle composition using the subcritical water emulsification technology will be described below with reference to Figure.

Figure is a view for explaining a manufacturing step of a monomer particle dispersion liquid using a supercritical water supply device. A supercritical water supply device "SFW-E40S" in a supercritical water emulsification apparatus (available from AKICO) shown in Figure, which can supply subcritical water to supercritical water, is used for subcritical water emulsification processing. This apparatus includes a "mixing portion 100" formed by inflow paths for two raw materials and a mixer in which the liquids that have flowed in merge, a "supercritical/subcritical process portion 200" arranged next to the mixing portion 100 and configured to change water into hot water in a supercritical state or a subcritical state, a mixing portion 300 that mixes a liquid output from the "supercritical/subcritical process portion 200" and an emulsifier, and a "cooling portion 400" that rapidly cools the liquid in the subcritical state, which is mixed with the emulsifier, at a speed of about 150°C/sec, thereby obtaining an emulsion with a particle size in nanometer.

Here, the steps of the manufacturing method (i) of the resin particle composition correspond to the steps from the mixing portion 100 located at the most upstream position to the subcritical process portion 200. The steps of the manufacturing method (ii) correspond to the steps from the mixing portion 300 located on the downstream side of the subcritical process portion 200 to the cooling portion 400. Note that the steps of the manufacturing method (iii) are based on a general polymerization method, and a detailed description thereof will be omitted.

In the present invention, a nonionic emulsifier having a cloud point is added to a liquid output from the supercritical/subcritical process portion 200. The present invention obtains a dispersion liquid of a polymerizable monomer generated by finely emulsifying in water from a liquid raw material containing a polymerizable monomer in which a marker substance is dissolved under milder conditions. As the conditions in the supercritical/subcritical process portion 200, the water temperature is set to a high temperature of 250°C to 370°C before the critical temperature, and the pressure is set to a high pressure of 25 MPa at which subcritical water is obtained. Hence, the supercritical/subcritical process portion 200 will be referred to as a "subcritical process portion 200" as shown in Figure hereinafter. A supplementary explanation will be made here, concerning subcritical water emulsification.

### (Explanation of Subcritical Water Emulsification)

It is known that the relative permittivity of water largely lowers near the critical point. For example, it is known that the relative permittivity of water is as low as that of methanol near 200°C as low as, that of acetone near 300°C, and as low as that of an organic solvent such as ethyl acetate near the critical point, and water is compatible with many oils and dissolvable. In particular, a technique of, to use many organic oils, performing the following three steps (dissolving, precipitation, and emulsification/stabilization steps) in continuous flow method in a subcritical region lower than the critical point of water and preparing a micro emulsion in a short time is a micro emulsification technique. A technique of emulsifying an oil into fine particles in nanometer in a shorter time (more specifically, in seconds) than in the continuous flow method (1 pass) without stirring is the micro emulsification technique. As described above, since fine particles in nanometer can be generated in a short time, precipitation/drop to the outside of oil droplets never occurs. The present invention can produce even and fine monomer oil droplets that can hardly be implemented by a conventional technique.
(1) Dissolving step -> An oil component is temporarily dissolved and evenly dispersed in subcritical water
(2) Precipitation step -> The temporarily dissolved oil component is precipitated by rapid cooling
(3) Emulsification step -> The precipitated fine particles are stabilized using an emulsifier (unification is suppressed)

By the steps (1) to (3), fine oil droplet particles in nanometer can be obtained by the bottom-up method.

### <Manufacturing of Monomer Particle Dispersion Liquid>

Table 1 shows the raw material compositions and evaluation results of monomer particle dispersion liquids of Manufacturing Examples 1 to 11. An average oil droplet diameter (nm) and polydispersity index (p.d.) in Table 1 are values measured by a particle analyzer FPAR-1000 available from Otsuka Electronics. In precipitation evaluation of a fluorescent dye or the like (including an AIE dye), the monomer particle dispersion liquids were irradiated with UV light, and precipitation of the fluorescent dye or the like was confirmed. In Table 1, the precipitation amount of the fluorescent dye or the like increases in the order of "none", "small", and "slightly large" in the field of precipitation of the fluorescent dye or the like. The monomer particle dispersion liquid of Manufacturing Example 11 did not contain a fluorescent dye or the like, and was excluded from the target of precipitation evaluation of the fluorescent dye or the like (indicated by "-"). Note that a polymerizable monomer includes a monomer of a main component and a monomer including a functional group in Table 1. A fluorescent dye amount (with respect to a monomer) indicates the composition amount (wt%) of the fluorescent dye or the like with respect to 100 mass% of a polymerizable monomer used in manufacturing of a monomer particle dispersion liquid. Table 2 shows the raw material compositions and test results of resin particle compositions of Examples 1 to 5 and Comparative Examples 1 to 8.

**Table 1]**

| | Type of fluorescent dye or the like | Fluorescent dye amoun t (with respec t to mono mer: wt%) | Monomer of main component | Monomer particles including functional group | Oil droplet producti on apparatus | Emulsification time (min) | Average oil droplet size (nm ) | Polydis persity index (p.d.) | Precipi tation of fluores cent dye or the like |
|---|---|---|---|---|---|---|---|---|---|
| Manufacturing example 1 | anthra cene | 10 | ST | mAA c | supercritical water emulsification apparatus | 3 | 150 | 0.025 | none |
| Manufacturing example 2 | HPS | 5 | ST | mAA c | supercritical water emulsification apparatus | 3 | 160 | 0.032 | none |
| Manufacturing example 3 | anthracene | 1 | MMA | AM | supercritical water emulsification apparatus | 3 | 90 | 0.032 | none |
| Manufacturing example 4 | HPS | 0.5 | MMA | mAA c | supercritical water emulsification apparatus | 3 | 450 | 0.044 | none |
| Manufacturing example 5 | HPS | 1 | ST | 2HE MA | supercritical water emulsification apparatus | 3 | 110 | 0.021 | none |
| Manufacturing example 6 | anthracene | 1 | ST | 2HE MA | Homodisper | 10 | 430 0 | 0.711 | slightly large |
| Manufacturing example 7 | HPS | 1 | ST | mAA c | high-pressure homage nizer | 30 | 120 | 0.085 | slightly large |
| Manufacturing example 8 | HPS | 0.5 | MMA | mAA c | ultrasonic homoge nizer | 20 | 190 | 0.196 | slightly large |
| Manufacturing example 9 | anthracene | 1 | MMA | mAA c | high-pressure homagenizer | 3 | 420 | 0.247 | small |
| Manufacturing example 10 | HPS | 1 | ST | AM | ultrasonic homogenizer | 3 | 540 | 0.361 | small |
| Manufacturing example 11 | - | - | ST | mAA c | high-pressure homogenizer | 30 | 130 | 0.096 | - |

### (Explanation of Abbreviations)

HPS: hexaphenylsilole
ST: styrene
MMA: methyl methacrylate
mAAc: methacrylic acid
AM: acrylamide
2HEMA: 2-hydroxyethyl methacrylate
The mass part of a monomer of a main component: the mass part of a monomer including a functional group = 90 : 10 (common to Manufacturing Examples 1 to 11)
homodisper (MARKII Model 2.5 available from Primix, stirring condition: rotation speed 5,000 rpm)
ultrasonic homogenizer (UX-600 available from Mitsui Electric Seiki, stirring condition: frequency 20 KHz)
high-pressure homogenizer (LAB2000 available from SMT, stirring condition: 5 cycles under a pressure of 200 MPa)

**Table 2]**

| | Type of monom er particle dispersion liquid | Crosslinkable monomer | Amount of crosslinkable monomer (with respect to monomer: wt%) | Type of initiator | Average particle size (nm ) | Marker substance absorption process | | Existence of fluorescent dye or the like outside resin particles | Fluorescence intensity (after water washing) | Fluorescence intensity (THF dispersion liquid) |
|---|---|---|---|---|---|---|---|---|---|---|
| | | | | | | Type of fluorescent dye or the like | Fluorescent dye amount (with respect to resin particles: wt%) | | | |
| Exam ple 1 | Manufa cturing exampl e 1 | DVB | 10 | AI BN | 160 | none | | none | ○ | ○ |
| Exam ple 2 | Manufa cturing exampl e 2 | DVB | 20 | KP S | 150 | none | | almo st none | ⊚ | ○ |
| Exam ple 3 | Manufa cturing exampl e 3 | TMP | 10 | KP S | 100 | none | | almo st none | ○ | ○ |
| Exam ple 4 | Manufa cturing exampl e 4 | TMP | 20 | AI BN | 470 | none | | none | ○ | ○ |
| Exam ple 5 | Manufa cturing exampl e 5 | DVB | 30 | AI BN | 120 | none | | none | ⊚ | ○ |
| Comp arative examp le 1 | Manufa cturing exampl e 6 | - | 0 | KP S | 200 | none | | large | × | × |
| Comp arative examp le 2 | Manufa cturing exampl e 7 | - | 0 | AI BN | 130 | none | | slight ly large | Δ | × |
| Comp arative examp le 3 | Manufa cturing exampl e 8 | - | 0 | AI BN | 190 | none | | slight ly large | Δ | × |
| Comp arative examp le 4 | Manufa cturing exampl e 9 | - | 0 | KP S | 280 | none | | large | × | × |
| Comp arative examp le 5 | Manufa cturing exampl e 10 | - | 0 | AI BN | 450 | none | | slight ly large | Δ | × |
| Comp arative examp le 6 | Manufa cturing exampl e 7 | DVB | 20 | AI BN | 160 | none | | slight ly large | Δ | Δ |
| Comp arative examp le 7 | Manufa cturing exampl e 11 | - | 0 | AI BN | 130 | HPS | 1 | Little | Δ | × |
| Comp arative examp le 8 | Manufa cturing exampl e 11 | DVB | 20 | AI BN | 150 | HPS | 1 | Large | × | × |

### (Explanation of Abbreviations)

DVB: divinylbenzene
TMP: trimethylolpropane trimethacrylate
AIBN: 2,2'-azobis(isobutyronitrile)
KPS: potassium persulfate
HPS: hexaphenylsilole
A formula for converting the amount (wt%) of a crosslinkable monomer into a mass part: mass part = 100X/(1 - X), here X indicates wt%

### <Manufacturing Example 1: Preparation of Monomer Particle Dispersion Liquid>

Based on Table 1, ion exchange water, a marker substance dissolved polymerizable monomer liquid (anthracene: styrene: methacrylic acid (= 10 parts: 90 parts: 10 parts)), and a dilute emulsifier solution (22.5% dilute aqueous solution of NOIGEN XL-100 (product name, available from DKS, polyoxyalkylene branched decyl ether, cloud point = 79°C, HLB 14.7)) were prepared as liquid raw materials.

The ion exchange water and the marker substance dissolved polymerizable monomer liquid (a liquid mixture of anthracene, styrene, and methacrylic acid) were supplied as the liquid raw materials from the inflow paths of the supercritical water emulsification apparatus shown in Figure at rates of 29.5 ml/min and 4.3 ml/min, respectively. In the subcritical process portion, the marker substance dissolved polymerizable monomer liquid was dissolved in the ion exchange water under high-temperature/high-pressure conditions that water changed to subcritical water at 300°C. Next, the dilute emulsifier solution (a nonionic emulsifier solution having a cloud point) was added to the liquid output from the subcritical process portion at a rate of 5.2 ml/min at an early stage when monomer particles began to be formed, and the resultant liquid was cooled in the cooling portion, thereby obtaining a monomer particle dispersion liquid made of fine monomer particles having a marker substance dissolved polymerizable monomer liquid concentration of 10% and an average particle size of 150 nm.

The particle size distribution of the obtained monomer particles exhibited a sharp normal distribution, and it could be confirmed that it was a dispersion liquid made of even fine particles. The average particle size and the particle size distribution of the obtained monomer particles were obtained by a dynamic light scattering method. The measurement of the average particle size (diameter D50) by the dynamic light scattering method was done at 25°C using FPAR-1000 (product name, available from Otsuka Electronics) after the monomer particle dispersion liquid was diluted by water to an appropriate concentration. In addition, when the obtained monomer particle dispersion liquid was irradiated with a UV lamp, and the diluted state of the marker substance was observed, precipitation of the marker substance was not observed. It was thus confirmed that the marker substance was incorporated in the monomer particles.

### <Manufacturing Examples 2 to 11: Preparation of Monomer Particle Dispersion Liquid>

The monomer particle dispersion liquids of Manufacturing Examples 2 to 5 were prepared based on the same manufacturing method as in Manufacturing Example 1 and Table 1, and a detailed description thereof will be omitted. On the other hand, the monomer particle dispersion liquids of Manufacturing Examples 6 to 11 were prepared based on the raw material compositions in Table 1 using oil droplet production apparatuses (a homodisper, a high-pressure homogenizer, and an ultrasonic homogenizer) different from Manufacturing Examples 1 to 5.

### <Example 1: Preparation of Resin Particle Composition>

In a 300-ml four-neck round bottom flask with a stirrer, a thermometer, and a reflux condenser, 100 g of a monomer particle dispersion liquid containing anthracene and having an average particle size of 150 nm, which was obtained in Manufacturing Example 1, 10 wt% DVB as a crosslinkable monomer with respect to 100 mass% of a total polymerizable monomer (converted to 1.1 g in weight), and 0.5 g of AIBN as a polymerization initiator were added, and stirred at 25°C for 60 min at a rotation speed of 170 rpm under an atmospheric pressure while performing nitrogen substitution. Then, 120.9 g of ion exchange water were added, and the resultant liquid was stirred at an inner temperature of 75°C for 6 hrs and cooled after a polymerization process, thereby obtaining a resin particle composition containing anthracene and having a concentration of 5% and an average particle size of 160 nm.

The measurement of the average particle size (D50) and the particle size distribution of the resin particle composition obtained by polymerization was done by the dynamic light scattering method, as in the manufacturing examples. In addition, when the obtained resin particle composition was irradiated with a UV lamp, and the diluted state of the marker substance was observed, precipitation of the marker substance was not observed. Furthermore, when the obtained fine resin particle composition was observed using an electron microscope, no aggregate other than the resin particle composition was observed, and it was confirmed that the marker substance was incorporated in the resin particles.

### <Examples 2 to 5 and Comparative Examples 1 to 8: Preparation of Resin Particle Composition>

The resin particle compositions of Examples 2 to 5 and Comparative Examples 1 to 8 were prepared based on the liquid raw materials in Table 2 and the same manufacturing method as in Example 1, and a detailed description thereof will be omitted.

### <Test Method>

Performance evaluation was performed by conducting tests of Test Examples 1 to 3 below using the resin particle compositions of Examples 1 to 5 and Comparative Examples 1 to 8. Note that for the resin particle compositions of Comparative Examples 7 and 8, the tests of Test Examples 1 to 3 were performed after the following marker substance absorption process was performed.

### (Explanation of Marker Substance Absorption Process)

This step aims at confirming whether the fluorescence intensity improves when the resin particle compositions of Comparative Examples 7 and 8 absorb the marker substance. The marker substance (to be referred to as HPS: hexaphenylsilole hereinafter) was dissolved in tetrahydrofuran (to be also referred to as THF hereinafter), thereby producing a THF dispersion liquid in which HPS having a concentration of 1 g/L was dissolved. The THF dispersion liquid in an amount with which the amount of HPS became 1 wt% was mixed with the resin particles of the produced resin particle dispersion liquid, and stirring was performed for 5 min, thereby causing the resin particle composition to absorb HPS. After that, the obtained dispersion liquid was subjected to ultracentrifuge to make the resin particles sink. After a supernatant was removed, pure water was added, and the particles were redispersed, thereby obtaining a resin particle dispersion liquid.

### (Test Example 1, Confirmation of Existence of Fluorescent Dye or the Like Outside Resin Particles)

After the resin particle dispersion liquid was sampled and dried at 60°C for 1 hr, it was confirmed whether a fluorescent dye or the like (including an AIE dye) existed outside the resin particles using a scanning electron microscope (SEM) available from JEOL.

### (Evaluation Criteria)

None: no fluorescent dye or the like was confirmed outside the resin particles Almost none: a fluorescent dye or the like was rarely confirmed outside the resin particles
Little: a fluorescent dye or the like was confirmed a little outside the resin particles
Slightly large: a small amount of fluorescent dye or the like was confirmed outside the resin particles
Large: a large amount of fluorescent dye or the like was confirmed outside the resin particles

### (Test Example 2, Confirmation of Fluorescence Intensity (After Water Washing))

The obtained resin particle dispersion liquid was subjected to ultracentrifuge to make the resin particles sink. After a supernatant was removed, pure water was added, and the particles were redispersed. This operation was repeated three times. The resin particles sunk for the fourth time were diluted by pure water to produce a dispersion liquid with a concentration of 1 mg/mL. The dispersion liquid was captured using a fluorescence microscope BZ-X800 available from Keyence, and the fluorescence intensity was confirmed.

### (Evaluation Criteria)

⊚: very high, O: high, △: slightly high, ×: low

### (Test Example 3, Confirmation of Fluorescence Intensity (THF Dispersion Liquid))

The obtained resin particle dispersion liquid was subjected to ultracentrifuge to make the resin particles sink. After a supernatant was removed, pure water was added, and the particles were redispersed. This operation was repeated three times. The resin particles sunk for the fourth time were diluted by pure water to produce a dispersion liquid with a concentration of 10 mg/mL. The dispersion liquid was captured using a fluorescence microscope BZ-X800 available from Keyence, and the fluorescence intensity was confirmed.

### (Evaluation Criteria)

⊚: very high, O: high, △: slightly high, ×: low

### <Test Results>

Table 2 shows the test results of Examples 1 to 5 and Comparative Examples 1 to 8. Examples 1 to 5 exhibited excellent characteristics (solvent resistance and high fluorescence intensity) in Test Examples 1 to 3 as compared to Comparative Examples 1 to 8. In particular, Examples 1 to 5 had high fluorescence intensities in the solvent (THF). On the other hand, Comparative Examples 1 to 5 (a crosslinkable monomer was not contained) showed that the fluorescent dye or the like dropped to the outside of the resin particles, and the fluorescence intensity lowered. Comparative Example 6 (a crosslinkable monomer was contained) showed that the solvent resistance of the resin particles slightly improved but did not exhibited a fluorescence intensity sufficient for a practical use. Comparative Examples 7 and 8 (the marker substance was absorbed in the post-process) showed that the fluorescent dye or the like adhered to the surfaces of the resin particles easily dropped, and the fluorescence intensity lowered. According to the above-described test results, it is considered that manufacturing resin particles using the monomer particle dispersion liquid manufactured using the subcritical water emulsification technology of the present invention and the crosslinkable monomer contributes to improvement of the solvent resistance. It is thus considered that this can prevent the marker substance incorporated in the resin particles from leaking, and a high fluorescence intensity can be implemented.

As described above, the resin particle composition according to the present invention has notable effects of implementing an excellent solvent resistance and providing a high fluorescence intensity.

Note that it should be understood that it is difficult for a person skilled in the art to directly specify detailed structures and characteristics of the monomer particle dispersion liquid and the resin particles according to the present invention, and detailed information thereof is not yet clear. In addition, note that directly specifying detailed structures and characteristics of the monomer particle dispersion liquid and the resin particles according to the present invention is not realistic from the viewpoint that a person skilled in the art is required to make remarkably excessive economical expenses for excessive experiments and preparation of special measurement devices.

The invention is not limited to the foregoing embodiments, and various variations/changes are possible within the spirit of the invention.

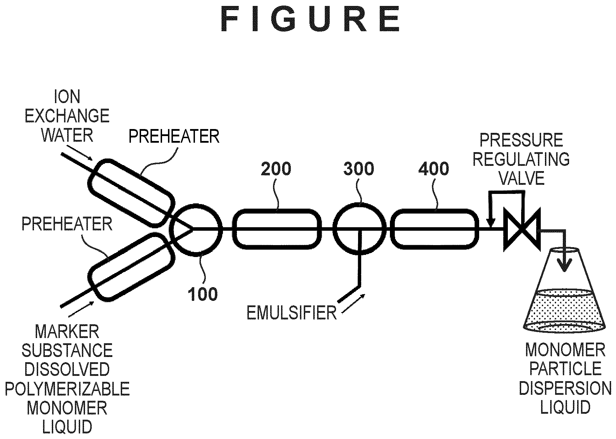

## Claims

1. A resin particle composition containing resin particles incorporating a marker substance,
wherein the resin particle composition is manufactured by a method comprising:
generating a mixture by mixing the marker substance and a polymerizable monomer using subcritical water (100, 200);
generating a monomer particle dispersion liquid by mixing and cooling the mixture and an emulsifier (300, 400); and
generating the resin particles by causing a polymerization reaction between the monomer particle dispersion liquid and a crosslinkable monomer,
wherein the polymerizable monomer contains at least one of an aromatic monomer, at least one of alkyl (meth)acrylates containing an alkyl group whose number of carbon atoms is 1 to 18 and cyclohexyl (meth)acrylates, and an alkyl (meth)acrylate containing a functional group,
the resin particles contain 40 to 95 mass% of the aromatic monomer and/or at least one of the alkyl (meth)acrylates containing an alkyl group whose number of carbon atoms is 1 to 18 and the cyclohexyl (meth)acrylates, 0 to 10 mass% of the alkyl (meth)acrylate containing the functional group, and 5 to 60 mass% of the crosslinkable monomer as constituent units with respect to 100 mass% of a total polymerizable monomer, and
the marker substance contains at least one of a fluorescent dye and an aggregation-induced emission dye,
the aromatic monomer includes at least one substance selected from the group consisting of styrene, methylstyrene, chlorostyrene, methoxystyrene, α-methylstyrene, p-nitrostyrene, ethylvinylbenzene, vinylnaphthalene, benzyl acrylate, benzyl methacrylate, phenylethyl acrylate, phenylethyl methacrylate, phenylpropyl acrylate, phenylpropyl methacrylate, phenylnonyl acrylate, and phenylnonyl methacrylate, and
the functional group includes at least one of a hydroxy group, a carboxy group, an amino group, and an amide group.

2. The resin particle composition according to claim 1, wherein the resin particles contain 0.1 to 15 mass% of the marker substance with respect to 100 mass% of the total polymerizable monomer.

3. The resin particle composition according to claims 1 or 2, wherein an average particle size of the resin particles is 50 to 500 nm, and
the average particle size is measured by a dynamic light scattering method.

4. The resin particle composition according to any one of claims 1 to 3, wherein the crosslinkable monomer includes at least one substance selected from the group consisting of allyl methacrylate, ethyleneglycol dimethacrylate, ethyleneglycol diacrylate, butanediol diacrylate, butanediol dimethacrylate, neopentyl glycol dimethacrylate, hexanediol dimethacrylate, triethyleneglycol dimethacrylate, tetraethyleneglycol dimethacrylate, trimethylolpropane trimethacrylate, pentaerythritol tetramethacrylate, and divinylbenzene.

5. The resin particle composition according to any one of claims 1 to 4, wherein the emulsifier is a nonionic emulsifier having a cloud point.

6. The resin particle composition according to any one of claims 1 to 5, wherein the fluorescent dye includes at least one substance selected from the group consisting of merocyanine, perylene, acridine, anthracene, luciferin, pyranine, stilbene, rhodamine, coumarin, 4-(dicyanomethylene)-2-methyl-6-(4-dimethyl aminostyryl)-4H-pyran (DCM), pyrromethene, fluorescein, umbelliferone, and derivatives thereof, and
the aggregation-induced emission dye includes at least one substance selected from the group consisting of a silole ring-containing compound and derivative, a hydrocarbon aromatic-based compound and derivative, a heteroaromatic-based compound and derivative, and a rhodamine-based compound and derivative.

7. A test kit comprising a resin particle composition defined in any one of claims 1 to 6, which binds to a marker substance in a specimen.

8. A manufacturing method of a resin particle composition containing resin particles incorporating a marker substance, comprising:
generating a mixture by mixing the marker substance and a polymerizable monomer using subcritical water (100, 200);
generating a monomer particle dispersion liquid by mixing and cooling the mixture and an emulsifier (300, 400); and
generating the resin particles by causing a polymerization reaction between the monomer particle dispersion liquid and a crosslinkable monomer,
wherein the polymerizable monomer contains at least one of an aromatic monomer, at least one of alkyl (meth)acrylates containing an alkyl group whose number of carbon atoms is 1 to 18 and cyclohexyl (meth)acrylates, and an alkyl (meth)acrylate containing a functional group,
the resin particles contain 40 to 95 mass% of the aromatic monomer and/orat least one of the alkyl (meth)acrylates containing an alkyl group whose number of carbon atoms is 1 to 18 and the cyclohexyl (meth)acrylates, 0 to 10 mass% of the alkyl (meth)acrylate containing the functional group, and 5 to 60 mass% of the crosslinkable monomer as constituent units with respect to 100 mass% of a total polymerizable monomer, and
the marker substance contains at least one of a fluorescent dye and an aggregation-induced emission dye,
the aromatic monomer includes at least one substance selected from the group consisting of styrene, methylstyrene, chlorostyrene, methoxystyrene, α-methylstyrene, p-nitrostyrene, ethylvinylbenzene, vinylnaphthalene, benzyl acrylate, benzyl methacrylate, phenylethyl acrylate, phenylethyl methacrylate, phenylpropyl acrylate, phenylpropyl methacrylate, phenylnonyl acrylate, and phenylnonyl methacrylate, and
the functional group includes at least one of a hydroxy group, a carboxy group, an amino group, and an amide group.
